# EUROPÄISCHE PATENTANMELDUNG

(11) **EP 1 652 545 A1**
(43) Veröffentlichungstag der Anmeldung: **03.05.2006**
(21) Anmeldenummer: 04025784.2
(22) Anmeldetag: 29.10.2004
(51) Int. Cl.: A61M 5/32, A61M 25/00, A61B 1/00, A61K 9/00, B29C 45/00

(54) **Oberfläche für den Kontakt mit menschlichem, tierischem oder künstlichem Gewebe**

(71) Anmelder: Universität Duisburg-Essen, 45141 Essen (DE)
(72) Erfinder: Büscher, Robin, Dipl.-Ing., 46049 Oberhausen (DE); Zietsch, Christian, Dipl.-Ing., 45475 Mühlheim a. d. Ruhr (DE)
(74) Vertreter: Gesthuysen, von Rohr & Eggert

(57) **Zusammenfassung**

Es wird eine Oberfläche mit mikroskopischen Vorsprüngen bzw. Vertiefungen zur Verringerung der Reibung zwischen der Oberfläche und menschlichem, tierischem oder künstlichem Gewebe, insbesondere Haut, vorgeschlagen. Die Oberfläche ist insbesondere für medizinische Produkte einsetzbar.

## Beschreibung

Die vorliegende Erfindung betrifft eine Oberfläche gemäß dem Oberbegriff des Anspruchs 1, ein medizinisches Produkt gemäß dem Oberbegriff des Anspruchs 9 sowie eine Verwendung einer derartigen Oberfläche.

Bekannte medizinische bzw. chirurgische Nadeln oder Kanülen bestehen aus Metall und weisen eine polierte bzw. nicht strukturierte Oberfläche auf. Beim Einstechen in die Haut oder sonstiges Gewebe treten verhältnismäßig hohe Reibungskräfte auf, so daß das Einstechen verhältnismäßig hohe Kräfte erfordert und von dem Patienten oftmals als unangenehm empfunden wird. Bisher werden zur Verringerung dieser Reibungskräfte möglichst glatte Oberflächen eingesetzt.

Eine Mikrostrukturierung von Oberflächen ist grundsätzlich bereits bekannt. Implantate werden beispielsweise mit einer porösen, mikrostrukturierten Oberfläche versehen, um ein Einwachsen in Knochen zu verbessern, wie in der US 6,576,014 B2 offenbart. Aus der DE 101 57 315 C1 ist es bekannt, eine mikrostrukturierte Oberfläche als Lagerfläche bei einem Implantat einzusetzen. Die DE 199 37 707 A1 offenbart Werkzeug-Oberflächen mit definierter Mikrostruktur, die eine verringerte Adhäsionsneigung aufweisen, so daß eine unerwünschte Belagbildung an Vorrichtungen bei der Verarbeitung von unterschiedlichen Materialien, insbesondere beim Extrudieren von Kunststoff, verringert wird. Die DE 100 20 877 C1 offenbart die Mikrostrukturierung einer Oberfläche durch Prägen.

Der vorliegenden Erfindung liegt die Aufgabe zugrunde, eine Oberfläche, ein medizinisches Produkt sowie eine Verwendung einer derartigen Oberfläche anzugeben, wobei die Reibung - insbesondere die Gleitreibung und/oder Haftreibung - gegenüber menschlichem, tierischem oder künstlichem Gewebe verringert oder minimal ist.

Die obige Aufgabe wird durch eine Oberfläche gemäß Anspruch 1, ein medizinisches Produkt gemäß Anspruch 9 oder eine Verwendung gemäß Anspruch 11 gelöst. Vorteilhafte Weiterbildungen sind Gegenstand der Unteransprüche.

Eine grundlegende Idee der vorliegenden Erfindung liegt darin, daß die Oberfläche durch mikroskopische Vorsprünge bzw. Vertiefungen zur Verringerung der Reibung zwischen der Oberfläche und menschlichem, tierischem oder künstlichem Gewebe mikrostrukturiert ist. Im Gegensatz zu den sonst üblichen, glatten Oberflächen, die insbesondere poliert sind, kann die vorschlagsgemäße Mikrostrukturierung zu einer wesentlichen Verringerung der Reibung zwischen der Oberfläche und dem Gewebe führen. Dies gilt insbesondere im zumindest im wesentlichen trockenen Zustand von Oberfläche und Gewebe.

Unter "Mikrostrukturierung" sind bei der vorliegenden Erfindung Vorsprünge, Vertiefungen oder generell Oberflächenstrukturen in einem Größenbereich von etwa 1 µm bis etwa 1000 µm zu verstehen. Jedoch können die Strukturen gegebenenfalls auch noch kleiner bzw. feiner sein.

Weitere Vorteile, Merkmale, Eigenschaften und Aspekte der vorliegenden Erfindung ergeben sich aus der nachfolgenden Beschreibung bevorzugter Ausführungsbeispiele anhand der Zeichnung. Es zeigt:
- Fig. 1: eine schematische Darstellung eines vorschlagsgemäßen medizinischem Produkts in Form eines medizinischen oder chirurgischen Instruments mit einer vorschlagsgemäßen mikrostrukturierten Oberfläche;
- Fig. 2: eine ausschnittsweise, vergrößerte Schnittdarstellung der mikrostrukturierten Oberfläche;
- Fig. 3: eine schematische Darstellung eines vorschlagsgemäßen medizinischen Produkts in Form eines Mittels zur oralen oder analen Einnahme mit der strukturierten Oberfläche;
- Fig. 4: eine schematische Darstellung eines vorschlagsgemäßen Bedienelementes mit der strukturierten Oberfläche;
- Fig. 5: eine rasterelektronenmikroskopische Draufsicht der mikrostrukturierten Oberfläche; und
- Fig. 6: eine rasterelektronenmikroskopische Seitenansicht der mikrostrukturierten Oberfläche.

In den Figuren werden für gleiche oder ähnliche Teile die selben Bezugszeichen verwendet, wobei entsprechende oder ähnliche Vorteile und Eigenschaften erreicht werden, auch wenn eine wiederholte Beschreibung aus Vereinfachungsgründen weggelassen ist.

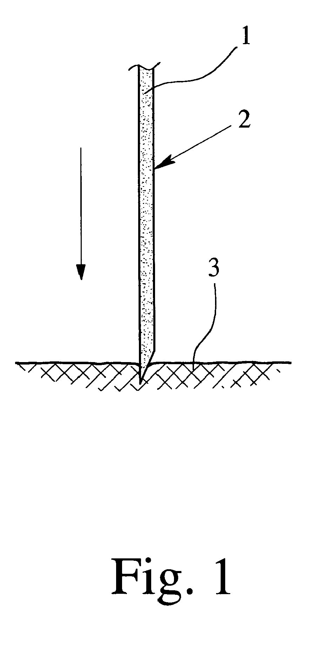

Fig. 1 zeigt in einer sehr schematischen Darstellung eine vorschlagsgemäße Oberfläche 1 eines medizinischen Produkts in Form eines medizinischen oder chirurgischen Instruments 2, das beim Darstellungsbeispiel als Nadel oder Kanüle ausgebildet ist. Die Oberfläche 1 kommt mit menschlichem, tierischem oder ggf. künstlichem Gewebe, insbesondere Haut 3, in Kontakt, beispielsweise beim Einstechen, wie in Fig. 1 durch den Pfeil angedeutet.

Die Oberfläche 1 besteht insbesondere aus Metall. Bisher ist es üblich, sehr glatte, insbesondere polierte Metalloberflächen einzusetzen, um einen vermeintlich geringen Widerstand beim Einstechen oder bei einem sonstigen Kontakt gegenüber der Haut 3 zu bilden. Des weiteren werden bisher glatte Oberflächen aus hygienischen Gründen bevorzugt, da diese wenig anfällig für die Aufnahme von Verunreinigungen oder dergleichen sind.

Die vorliegende Erfindung geht jedoch einen anderen Weg. Die Oberfläche 1 ist nämlich zur Verringerung der Reibung zwischen der Oberfläche 1 und menschlichem, tierischem oder künstlichem Gewebe, insbesondere der Haut 3, mikrostrukturiert.

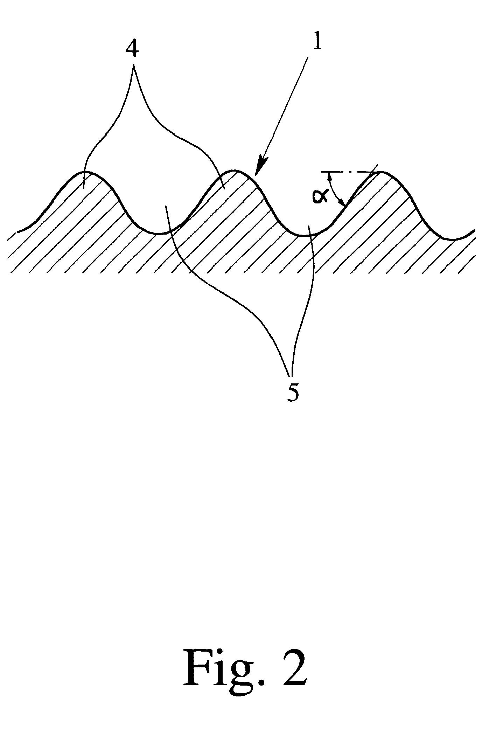

Fig. 2 zeigt in einer ausschnittsweisen, vergrößerten Schnittdarstellung die Oberfläche 1 mit mikroskopischen Vorsprüngen 4 und Vertiefungen 5. Versuche haben ergeben, daß die Mikrostrukturierung überraschenderweise zu einer Verringerung der Reibung - insbesondere der Haftreibung und/oder Gleitreibung - zwischen der Oberfläche 1 und menschlichem, tierischem oder künstlichem Gewebe, insbesondere Haut 3, führt.

Die Vorsprünge 4 oder Vertiefungen 5 sind vorzugsweise kugel-, halbkugel-, kegel- oder pyramidenförmig ausgebildet.

Die Vorsprünge 4 oder Vertiefungen 5 weisen vorzugsweise einen mittleren Abstand und/oder einen mittleren Durchmesser von etwa 10 bis 60 µm insbesondere im wesentlichen 20 bis 40 µm, auf.

Die Vorsprünge 4 oder Vertiefungen 5 weisen insbesondere eine mittlere Höhe bzw. Tiefe von etwa 5 bis 20 µm, insbesondere im wesentlichen 10 bis 15 µm, auf.

Die Vorsprünge 4 oder Vertiefungen 5 weisen vorzugsweise einen mittleren Neigungswinkel α gegenüber der makroskopischen Oberflächenkontur von etwa 20 bis 50°, insbesondere im wesentlichen 25 bis 35°, auf.

Die Flächendichte der Vorsprünge 4 oder Vertiefungen 5 beträgt vorzugsweise etwa 500 bis 5000/mm².

Die Oberfläche 1 besteht vorzugsweise aus Metall. Jedoch kann die Oberfläche 1 auch aus Glas, Keramik oder Kunststoff bestehen.

Die Mikrostrukturierung der Oberfläche 1 ist vorzugsweise durch insbesondere elektrochemisches Ätzen, Sintern, Gießen, Spritzgießen, Prägen, Laserbearbeitung oder eine sonstige geeignete Bearbeitung hergestellt. Beispielsweise kann die mikrostrukturierte Oberfläche 1 durch MIM (metal injection moulding), durch CIM (ceramic injection moulding) oder sonstige sinter- und/oder pulvermetallurgische Verfahren hergestellt werden.

Wie bereits erläutert, handelt es sich bei dem medizinischen Produkt vorzugsweise um ein medizinisches oder chirurgisches Instrument 2, wie eine Nadel oder Kanüle. Jedoch kann es sich hierbei auch um ein sonstiges medizinisches oder chirurgisches Instrument 2, wie einen Katheter, ein Endoskop, ein Anuskop, ein Proktoskop, ein Sigmoidoskop, ein Shinkteroskop, ein Laparoskop, einen Uterusmanipulator, ein Scheidenspekula, eine Biopsiezange oder dergleichen, oder um ein sonstiges medizinisches Produkt handeln. Wesentlich ist jeweils, daß das medizinische Produkt eine Oberfläche 1 für den Kontakt mit menschlichem, tierischem oder künstlichem Gewebe aufweist und daß diese Oberfläche 1 im genannten Sinne mikrostrukturiert ist, um die Reibung zwischen der Oberfläche 1 und dem Gewebe zu verringern bzw. zu minimieren.

Beispielsweise werden in der Gynäkologie Instrumente 2 eingesetzt, bei welchen eine geringe Reibung gegenüber dem Gewebe ausschlaggebend ist. Besonders interessant sind hier die Applikationen bzw. Untersuchungen, z. B. für Abstriche, bei denen keine zusätzlichen Substanzen, wie Gleitmittel, verwendet werden dürfen. Die vorschlagsgemäße Lösung zur Reduzierung der Reibung führt insbesondere dazu, daß die Handhabung vereinfacht wird und/oder die Beeinträchtigungen des Patienten minimiert werden.

Die vorschlagsgemäße Lösung zur Minimierung der Reibung gegenüber dem Gewebe ist nicht nur für das Einstechen in die Haut 3 vorteilhaft, sondern generell einsetzbar, wenn es um den Kontakt zwischen der Oberfläche 1 und menschlichem, tierischem oder künstlichem Gewebe geht, um eine geringe Reibung dazwischen zu ermöglichen.

Gemäß einer anderen Ausführungsform ist das medizinisches Produkt als Mittel zur oralen, nasalen oder analen Einnahme bzw. Applikation, insbesondere als Pille, Dragee, Tablette, Kapsel, Zäpfchen oder dergleichen, mit einer definierten Oberfläche für den Kontakt mit menschlichem, tierischem oder künstlichem Gewebe ausgebildet. Bei dem medizinischen Produkt kann es sich generell um Mittel oder Trägersubstanzen zur Einnahme und/oder Applizierung in allen natürlichen und ggf. sogar künstlichen Körperöffnungen, wie Ohr, Nase, Mund, Auge, Harnröhre oder Darmausgang, oder sonstige Produkte handeln, die in Kontakt mit menschlichem, tierischem oder künstlichem Gewebe kommen bzw. stehen. Die Oberfläche ist dabei zur Verringerung oder Minimierung der Reibung gegenüber dem Gewebe im oben genannten Sinne mikrostrukturiert.

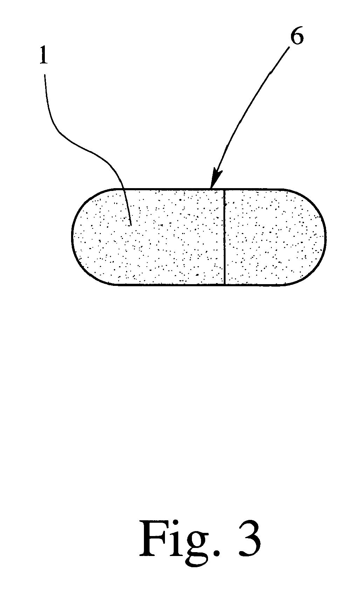

Fig. 3 zeigt beispielhaft ein vorschlagsgemäßes medizinisches Produkt bzw. Mittel 6 in Form einer Kapsel bzw. Tablette, wobei die Oberfläche 1 im oben genannten Sinne mikrostrukturiert ist. Aufgrund der Mikrostrukturierung ist die Kapsel bzw. Tablette leichter zu schlucken, da die Reibung, insbesondere bei trockenem Mund, gegenüber einer glatten Oberfläche deutlich verringert ist.

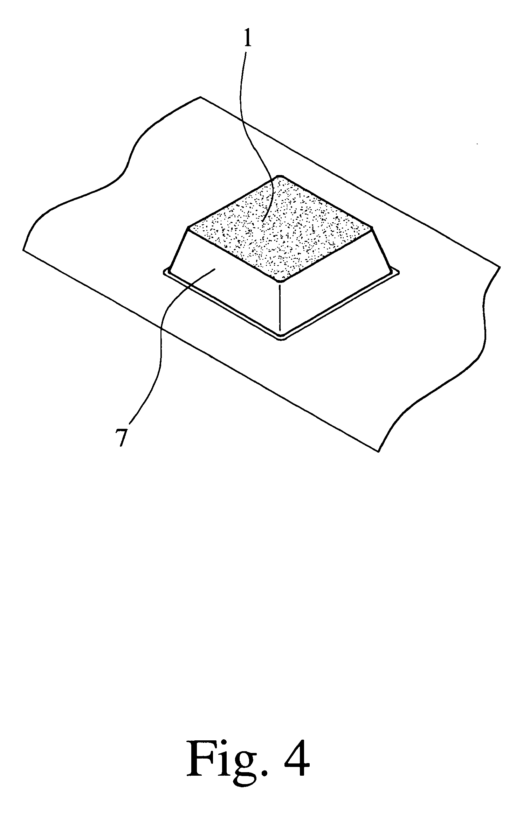

Fig. 4 zeigt ein vorschlagsgemäßes Bedienelement 7, insbesondere eine Taste, einen Schalter, ggf. aber auch einen Drehknopf oder dgl., wobei die Oberfläche 1 zumindest partiell im oben genannten Sinne mikrostrukturiert ist. Insbesondere ist die Oberfläche 1 in Bereichen mikrostrukturiert, in denen das Bedienelement 7 von einer nicht dargestellten Hand bzw. nicht dargestellten Fingern eines Benutzers erfaßt bzw. berührt wird. Aufgrund der Mikrostrukturierung ist wiederum die Reibung gegenüber der Haut verringert, so daß sich eine wesentliche angenehmere und/oder verbesserte Handhabung ergibt.

Zum Nachweis der Wirksamkeit der vorschlagsgemäßen Lösung wurden u.a. folgende Versuche durchgerührt:

Eine quaderförmige Probe (10 x 10 x 80 mm) aus rostfreiem Stahl wurde über eine Länge von etwa 20 mm mit der vorschlagsgemäßen Mikrostrukturierung versehen. Dies erfolgte vorzugsweise durch elektrochemisches Anätzen.

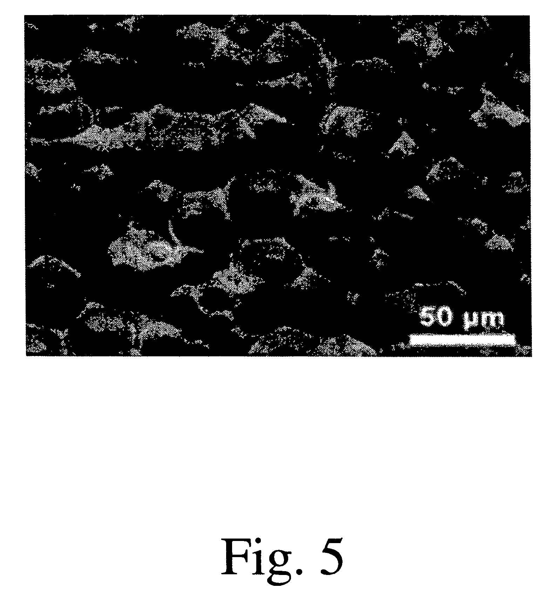

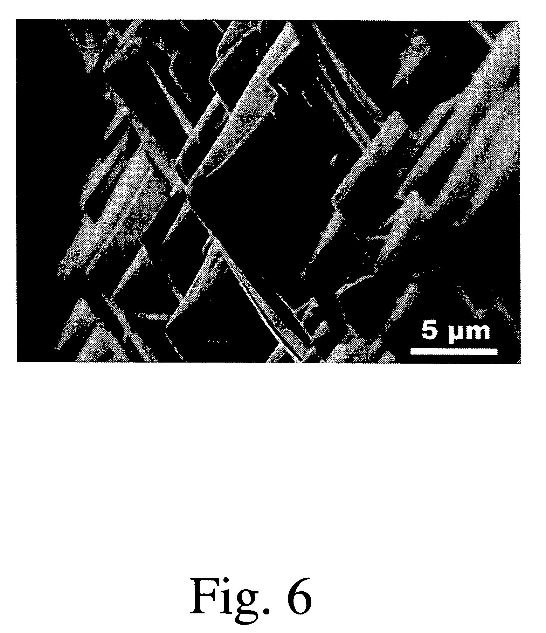

Bei dem Versuch wurde 450 ml H₂SO₄ mit 6 g CrCl₃ · 6 H₂O in 50 bis 100 ml H₂O eingesetzt. Die Kathode bestand aus Blei. Bei einer Spannung von 10 bis 15 V und einem maximalen Strom von 10 bis 12 A sowie bei einer Temperatur von 50 bis 80°C und mit einem Rührfisch bei 100 bis 300 U/min bzw. einer Strömung von 0,3 bis 0,6 m/s erfolgte ein Anätzen für 3 bis 10 min bzw. bis der Strom unter 0,02 A fiel. Die Fläche der angeätzten Oberfläche 1 betrug etwa 3000 mm². Fig. 5 und 6 zeigen einen durch das Anätzen vorschlagsgemäß mikrostrukturierten Oberflächenbereich in Draufsicht und Seitenansicht (es handelt sich um rasterelektronenmikroskopische Aufnahmen).

Die so behandelte Probe wies einerseits einen polierten Oberflächenbereich und andererseits den durch das vorgenannte Anätzen vorschlagsgemäß mikrostrukturierten Oberflächenbereich auf. Die polierten und die mikrostrukturierten Oberflächenbereiche waren jeweils paarweise auf gegenüberliegenden Seiten der Probe angeordnet.

Die Probe wurde in eine Versuchseinrichtung, eine servohydraulische Prüfmaschine, eingebaut, wobei die Probe zwischen gegenüberliegenden zylindrischen Metallkörpem, die mit unbehandelter Schweinehaut überzogen waren, eingeklemmt wurden. Die Probe wurde zwischen den Metallkörpern mit einem Hub vom 40 mm hin- und herbewegt, so daß die Schweinehaut jeweils etwa 20 mm mit einer polierten und anschließend etwa 20 mm mit einer strukturierten Oberfläche in Kontakt trat. Die Reibungskräfte, die Klemmkräfte und der Weg wurden kontinuierlich aufgezeichnet. Aus dem Quotienten der Reibungs- und Klemmkräfte (Normalkräfte) wurde der relevante Reibungskoeffizient µ berechnet.

Beim Übergang von dem strukturierten Oberflächenbereich in den polierten Oberflächenbereich nahmen die Reibungskraft und der Reibungskoeffizient immer um über 100 % zu. Bei einem Versuch nahm die Reibungskraft um etwa 138 % zu. Bei einem anderen Versuch nahm der Reibungskoeffizient um etwa 105 % zu.

Unbehandelte Schweinehaut verhält sich im wesentlichen wie menschliche Haut. Somit lassen sich die Versuchsergebnisse unmittelbar auf menschliche Haut übertragen.

Folglich führt die vorschlagsgemäße Mikrostrukturierung der Oberfläche 1 zu einer wesentlichen Verringerung der Reibung gegenüber den bisher üblicherweise eingesetzten glatten, insbesondere polierten Oberflächen.

Ergänzend ist darauf hinzuweisen, daß die vorliegende Erfindung auch auf anderen Gebieten einsetzbar ist. Insbesondere ist eine Verwendung der im oben genannten Sinne mikrostrukturierten Oberfläche 1 für künstliche Gewebe in einem sehr weiten Sinn möglich. Besonders bevorzugt ist auch eine Verwendung der mikrostrukturierten Oberfläche 1 für textiles Material, wie Fasern, Fäden, Garne, Wolle oder Textilien im allgemeinen. Hierbei wird wiederum die Reibung wesentlich verringert, so daß die Standzeit der Oberfläche bzw. damit versehener Führungselemente (wahlweise feststehend oder drehbar) erhöht und/oder das künstliche Gewebe bzw. die textilen Materialien, weniger beansprucht werden. Die vorliegende Erfindung erstreckt sich dementsprechend insbesondere auch auf Textilmaschinen, die im genannten Sinne mikrostrukturierte Oberflächen 1 zur Verringerung der Reibung aufweisen bzw. verwenden.

## Patentansprüche

1. Oberfläche (1), insbesondere eines medizinischen Produkts für den Kontakt mit menschlichem, tierischem oder künstlichem Gewebe, insbesondere Haut (3),
**dadurch gekennzeichnet,**
**daß** die Oberfläche (1) durch mikroskopische Vorsprünge (4) bzw. Vertiefungen (5) zur Verringerung der Reibung zwischen der Oberfläche (1) und dem Gewebe mikrostrukturiert ist.

2. Oberfläche nach Anspruch 1, **dadurch gekennzeichnet, daß** die Vorsprünge (4) oder Vertiefungen (5) kugel-, halbkugel-, kegel- oder pyramidenförmig ausgebildet sind.

3. Oberfläche nach Anspruch 1 oder 2, **dadurch gekennzeichnet, daß** die Vorsprünge (4) oder Vertiefungen (5) einen mittleren Abstand und/oder einen mittleren Durchmesser von etwa 10 bis 60 µm, vorzugsweise im wesentlichen 20 bis 40 µm, aufweisen.

4. Oberfläche nach einem der voranstehenden Ansprüche, **dadurch gekennzeichnet, daß** die Vorsprünge (4) oder Vertiefungen (5) eine mittlere Höhe bzw. Tiefe von etwa 5 bis 20 µm, vorzugsweise im wesentlichen 10 bis 15 µm, aufweisen.

5. Oberfläche nach einem der voranstehenden Ansprüche, **dadurch gekennzeichnet, daß** die Vorsprünge (4) oder Vertiefungen (5) einen mittleren Neigungswinkel gegenüber der makroskopischen Oberflächenkontur von etwa 20 bis 50°, vorzugsweise im wesentlichen 25 bis 30°, aufweisen.

6. Oberfläche nach einem der voranstehenden Ansprüche, **dadurch gekennzeichnet, daß** die Flächendichte der Vorsprünge (4) oder Vertiefungen (5) etwa 500 bis 5000/mm² beträgt.

7. Oberfläche nach einem der voranstehenden Ansprüche, **dadurch gekennzeichnet, daß** die Oberfläche (1) aus Metall, Glas, Keramik oder Kunststoff besteht.

8. Oberfläche nach einem der vorhergehenden Ansprüche, **dadurch gekennzeichnet, daß** die Oberfläche (1) durch Ätzen, Sintern oder sonstige pulvermetallurgische Herstellung, Gießen, Spritzgießen, Prägen oder Laserbearbeitung mikrostrukturiert ist.

9. Medizinisches Produkt mit einer Oberfläche (1) für den Kontakt mit menschlichem, tierischem oder künstlichem Gewebe, insbesondere Haut (3),
**dadurch gekennzeichnet,**
**daß** die Oberfläche (1) zur Verringerung der Reibung zum Gewebe gemäß einem der voranstehenden Ansprüche ausgebildet ist.

10. Medizinisches Produkt nach Anspruch 9, **dadurch gekennzeichnet, daß** das medizinische Produkt als medizinisches oder chirurgisches Instrument (2), insbesondere Nadel, Kanüle, Katheder, Endoskop, Anuskop, Proktoskop, Sigmoidoskop, Shinkteroskop, Laparoskop, Uterusmanipulator, Scheidenspekula oder Biopsiezange, oder als Mittel (6) zur Einnahme bzw. Applikation in natürliche oder künstliche Körperöffnungen, insbesondere zur oralen, nasalen oder analen Einnahme bzw. Applikation, vorzugsweise als Pille, Dragee, Tablette, Kapsel oder Zäpfchen, ausgebildet ist.

11. Verwendung einer nach einem der Ansprüche 1 bis 8 ausgebildeten Oberfläche (1) auf einem insbesondere gemäß dem kennzeichnenden Merkmal des Anspruchs 10 ausgebildeten medizinischen Produkt oder einem vorzugsweise manuell betätigbaren Bedienelement (7), wie Taste, Schalter oder Knopf, zur Verringerung der Reibung zwischen der Oberfläche (1) und menschlichem, tierischem oder künstlichem Gewebe, insbesondere Haut (3).
